(19) Europäisches Patentamt / European Patent Office / Office européen des brevets

(11) **EP 3 372 983 A1**

(12) **EUROPEAN PATENT APPLICATION**
published in accordance with Art. 153(4) EPC

(43) Date of publication:
**12.09.2018 Bulletin 2018/37**

(21) Application number: **16862120.9**

(22) Date of filing: **02.11.2016**

(51) Int Cl.:
**G01N 7/00** (2006.01)    **G01N 33/30** (2006.01)

(86) International application number:
**PCT/JP2016/082559**

(87) International publication number:
**WO 2017/078060 (11.05.2017 Gazette 2017/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.11.2015 JP 2015216189**
**24.08.2016 JP 2016163814**

(71) Applicant: **Imagineering, Inc.**
**Kobe-shi, Hyogo 6500-047 (JP)**

(72) Inventors:
- **IKEDA Yuji**
  **Kobe-shi**
  **Hyogo 650-0047 (JP)**
- **MAKITA Shinobu**
  **Kobe-shi**
  **Hyogo 650-0047 (JP)**

(74) Representative: **Haseltine Lake LLP**
**Lincoln House, 5th Floor**
**300 High Holborn**
**London WC1V 7JH (GB)**

(54) **VOID FRACTION MEASUREMENT METHOD**

(57)    An oil void fraction is measured by a simple way. A method of measuring an oil void fraction comprises a step of obtaining each oil void fraction of a plurality of sample oils, the void fraction thereof already-known, that are introduced into a closed space, compressing each sample oil with a predetermined pressure and measuring a volume change of the sample oil when compressed, and obtaining a calibration line for each sample oil, that is a linear function by connecting values represented by a product of the pressure at OkPa and the volume change plotted against the pressure when it is compressed to the predetermined pressure, a step of obtaining a value of a test oil sample having an unknown void fraction, that is represented by a product of the pressure at OkPa and the volume change plotted against the pressure when it is compressed to the predetermined pressure; and a step of determining the unknown void fraction of the test oil sample by comparing the value of the test sample oil to the calibration line of each sample oil.

FIG 1

EP 3 372 983 A1

## Description

### TECHNICAL FIELD

The present invention relates to a method of measuring a void fraction and a void fraction measuring system, specifically, a measuring method of a void fraction in oil such as a lubricant oil for an automobile.

### BACKGROUND ART

The method of measuring bubbles in liquid is typified by for example, the conductance method, the capacitance method, the wire-mesh method, the laser light cut-off type, the laser light scattering type (for example, non Patent Document 1), the probe technique (electric resistance detection method, photoelectric detection method) (for example, non Patent Document 2), the image analysis method (for example, non Patent Document 2), the resonation type weight measuring method, and the radiography method.

On the other hand, it is required to consider oil bulk modulus when the oil pressure driving device is designed. However, since the bulk modulus changes when bubbles are mixed into oil, the designing is required to be performed on considering this. There, the calculation method of the bulk modulus when the bubbles are mixed into the oil is suggested (non Patent Document 4).

### PRIOR ART DOCUMENTS

### PATENT DOCUMENT(S)

Non Patent Document 1: "SALD-7100 Nanoparticle Size Analyzer" (Shimaoka, Shimazu reviewal, separate print, 63th volume, the first, second number) (year 2006)
Non Patent Document 2: "Newly developed method for determining a touch position in sub-millimeter bubble/droplet measurement via a Single-Tip Optical fiber Probe" (Mizushima et al, Particulate Vol. 21, No. 73) (year 2012)
Non Patent Document 3: "Bubble behavior measurement in engine lubricant oil" (Kimura et al, Automobile Technology Association, Academic conference, separate print collection No. 147-08) (year 2008)
Non Patent Document 4: "On the bulk modulus of hydraulic fluid under entrained air condition" (Nakagawa et al, Bulletin of Faculty of Engineering of Toyama University, 27: 25-30) (year 1976)
Patent Document 1: Japanese Unexamined Patent Application Publication No. 2007-064820

### SUMARRY OF INVENTION

### PROBLEM TO BE SOLVED BY INVENTION

However, there are many cases that the device regarding the method of measuring the void fraction described as above is complex in system and the cost thereof is high expensive. Moreover, there are many commercially available measurement devices that they cannot respond to the measurement of the oil void fraction. For example, since the blue-violet light is selected as a light source for absorbing the oil in the measurement device by the laser light scattering type of for example Non Patent Document 1, it is not suitable for the oil void fraction measurement. On the other hand, since the measurement error occurs according to the oil sticking state into the probe distal end in light probe type of Non Patent Document 2, the calibration is required to be performed accordingly, and therefore, it is not suitable for the oil void fraction measurement.

Moreover, it is considered of the existence of a defect that a sufficient-size-light-amount-change cannot be detected when bubbles are separated from the wall surface (transparent) according to the method based on the image analysis described in Non Patent Document 3.

Moreover, various reviews with respect to an oil characteristic on the timing of the bubble mix into the oil as represented in Non Patent Document 4, for example are made in the oil-pressure-driving-device related art field. However, the document that refers to the void fraction calculation method has not been seen.

The present invention is made in view of above points, and the objective is to provide a method of measuring a void fraction included in oil and etc. in an easy way.

**MEANS FOR SOLVING THE ABOVE PROBLEMS**

A method of measuring an oil void fraction of the first present invention so as to solve the above problems comprises a step of introducing an oil having bubbles mixed therewith into a closed space, compressing the oil, and then measuring a bulk modulus of the oil having the bubbles, a step of referring to a predetermined oil bulk modulus of the oil which does not have the bubbles, which has been measured beforehand, and a step of determining the oil void fraction of the oil having the bubbles based on a ratio of the measured bulk modulus with respect to the referred bulk modulus.

A method of measuring an oil void fraction of the second present invention so as to solve the above problems comprises a step of obtaining each oil void fraction of a plurality of sample oils, the void fraction thereof already-known, that are introduced into a closed space, compressing each sample oil with a predetermined pressure and measuring a volume change of the sample oil when compressed, and obtaining a calibration line for each sample oil, that is a linear function by connecting values represented by a product of the pressure at OkPa and the volume change plotted against the pressure when it is compressed to the predetermined pressure, a step of obtaining a value of a test oil sample having an unknown void fraction, that is represented by a product of the pressure at OkPa and the volume change plotted against the pressure when it is compressed to the predetermined pressure, and a step of determining the unknown void fraction of the test oil sample by comparing the value of the test sample oil to the calibration line of each sample oil.

**EFFECT OF INVENTION**

According to the present invention, an oil void fraction can be measured in an easy way.

**BRIEF DESCRIPTION OF FIGURES**

Fig.1 shows a schematic structural view of an oil void fraction measurement system regarding the present embodiment.
Fig. 2 is a schematic view that shows a behavior of bubbles in oil.
Fig. 3 is a view that shows a calibration line so as to obtain the void fraction.
Fig. 4 is a concept view of a bubbles-mixed-engine-oil on the timing of an initial state and a compression-after state.
Fig. 5 is a graph that shows a relation between an in-cylinder pressure and a volume change amount.
Fig. 6 shows a graph that shows a relation of a product of a pressure and an air volume that the air behaves as a gas in test oil against the measured pressure of each test oil.
Fig. 7 shows a graph that shows a relation of the change rate of a product of a pressure and an air volume against the pressure of each test oil.
Fig. 8 shows a graph that shows a change of a term that is independent of pressure of a product of a pressure and the air volume against the pressure of each test oil.
Fig. 9 shows a graph that shows a relation of a product of a pressure and a volume change amount against the pressure when it is compressed to the predetermined pressure.
Fig. 10 shows a graph that shows a change of a term that is independent of pressure of a product of a pressure and the air volume against the pressure of each test sample, and it shows a calibration line of the second embodiment.

**EMBODIMENTS FOR IMPLEMENTING THE INVENTION**

In below, embodiments of the present invention are described in details based on figures. Note that, following embodiments are essentially preferable examples, and the scope of the present invention, the application, or the use is not intended to be limited.

**FIRST EMBODIMENT**

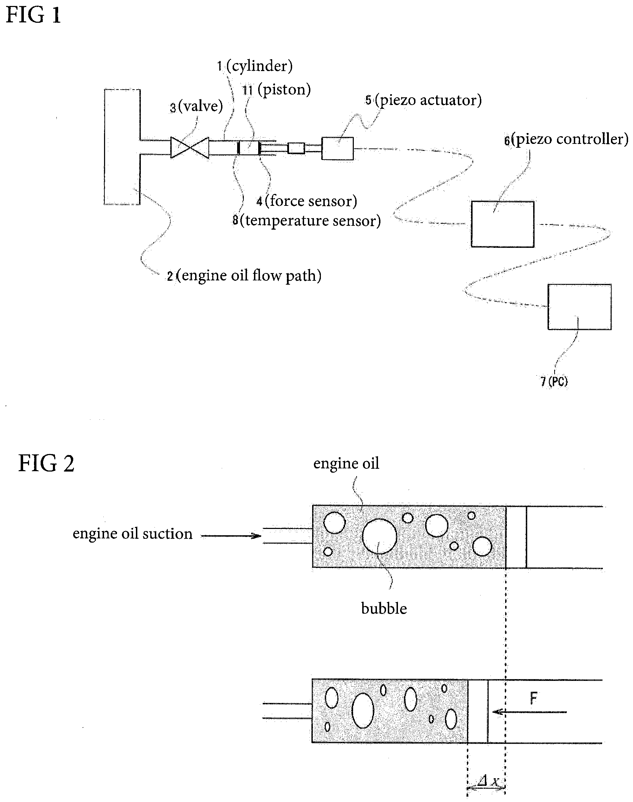

Referring to Fig. 1, an oil void fraction measurement system in which an oil void fraction measurement method of the present invention is applied, comprises a cylinder (injector) 1, a valve 3, a force sensor 4, a piezo actuator 5, a piezo controller 6, a personal computer 7, and a temperature sensor 8. The oil that flows through an engine oil flow path 2 is introduced into the cylinder 1, a piston 11 of the cylinder 1 is depressed by the piezo actuator 5, and then the oil inside the cylinder 1 is compressed in this measurement system. The oil void fraction is calculated by measuring a bulk modulus of oil having the bubbles on compression in this measurement system. The calculation method is described in below.

The valve 3 opens and closes the connection of the engine oil flow path 2 to the cylinder 1. The force sensor 4 measures a power size for depressing the piston 11. The piezo controller 6 generates an electric signal and etc. for driving the piezo actuator 5 based on instructions by control program that is stored in the personal computer 7. The temperature sensor 8 measures an oil temperature inside the cylinder 1. The power output from the force sensor 4, and the positional information output from the piezo actuator 5 are sent to the personal computer 7, and the above control program performs a control of the piezo actuator 5 based on these information.

Here, the bulk modulus "$K_{TB}$" of the oil having the bubbles at the temperature "T" can be calculated based on the mathematical formula 1.

[mathematical formula 1]

$$K_{TB} = \frac{1 + \left(\dfrac{P_0}{P}\right)\left(\dfrac{V_{g0}}{V_{l0}}\right)}{\dfrac{1}{K_{T0}} + \dfrac{1}{P}\left(\dfrac{P_0}{P}\right)\left(\dfrac{V_{g0}}{V_{l0}}\right)}$$

Here, an isothermal change in temperature is presupposed. "$P_0$" is an initial in-cylinder pressure, and here, atmospheric air pressure. "$V_{l0}$" is an initial engine oil volume, and "$V_{g0}$" is an initial total bubble volume. "$K_{T0}$" is an engine oil bulk modulus at the temperature "T" under the situation where the bulk is not mixed into, and here one measured beforehand is used. "P" is an in-cylinder pressure when the piston is pressed at the power "F". Note that, the derivation process of the mathematical formula 1 is expressed in the Non Patent Document 4, and therefore, the explanation thereof is omitted.

Moreover, if the in-cylinder volume "V" is considered to be sum up of the engine oil volume "V1" and the total bubble volume "Vg", and almost all the most compression/expansion of the gas/liquid two-phase fluid is deemed to be due to the gaseous phase compression/expansion (referring to Fig. 2), the mathematical formula 2 is established.

[mathematical formula 2]

$$PV_g = const. = P_0 V_{g0}$$

That is, if a displacement of the piston 11 "$\Delta x$" (referring to Fig. 2) is measured, a variation of the bubble volume "$V_G$" of the oil inside the cylinder 1 can be specified, and thereby, the pressure "P" of the mathematical formula 1 can be calculated.

Moreover, if the cylinder 1 is placed under the atmospheric air pressure and a state where the power "F" is not depressed to the piston 11 is made at an initial state for example, an initial in-cylinder pressure "$V_0$" (sum up of "$V_{l0}$" and "$V_{g0}$" abovementioned) can be specified. The power "F" is gradually depressed to the piston 11 from this state, and then it is difficult to press the piston 11 any further. The oil bubble volume at that time is deemed to be small sufficiently compared to the oil solution, and thereby, the value of "$V_{l0}$" can also be specified.

That is, if at least a positional information of the piston 11 at an initial pressure and a positional information of the piston 11 when the piston 11 cannot be pressed any further are transmitted to the personal computer 7, the personal computer 7 can calculate the bulk modulus "$K_{TB}$" of the oil having the bubbles based on the formula stored beforehand in the control program.

Moreover, in the present embodiment, a focus is put on the point that the oil compressibility can be expressed by an inverse number of an engine oil bulk modulus, a ratio of the engine oil compressibility "$\beta_{TB}$" inside the cylinder 1 at the temperature "T" when the in-cylinder pressure P is gradually changed, with respect to the engine oil compressibility "$\beta_{TO}$" at the temperature "T" at the state where the bubbles are not mixed into, is monitored (measured), and thereby, the void fraction is measured. Here, the ratio of the above compressibility "$\beta_{TB}/\beta_{TO}$" can be expressed by the mathematical formula 3.

[mathematical formula 3]

$$\frac{\beta_{TB}}{\beta_{T0}} = \frac{1 + K_{T0}\frac{1}{P}\left(\frac{P_0}{P}\right)\left(\frac{V_{g0}}{V_{l0}}\right)}{1 + \left(\frac{P_0}{P}\right)\left(\frac{V_{g0}}{V_{l0}}\right)}$$

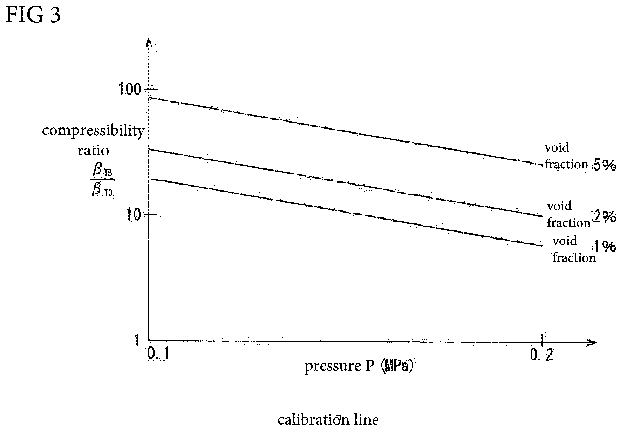

That is, while the inside of the cylinder 11 is controlled to become an isothermal change in temperature by depressing the piston 11 at an even power "F", the pressure "P" is gradually changed, and the ratio of the compressibility at that time is calculated (monitored) based on the mathematical formula 3. The void fraction is specified based on which one of calibration lines measured beforehand (referring to Fig. 3) a curve line expressing the relation between the ratio of the compressibility obtained as the result and the pressure matches with. Here, a calibration line is obtained by measuring the ratio of the oil compressibility in that the void fraction is already known. For example, already-known amount of air is introduced into the oil inside the cylinder 1 after introducing oil in the bubble-removed state into the cylinder 1, the position of the piston 11 is gradually displaced, and thereby, the pressure "P" is changed. The ratio of the compressibility "$\beta_{TB}/\beta_{T0}$" at that time becomes a calibration curve line. Note that, "$\beta_{T0}$" is an inverse number of "$K_{T0}$" as above-mentioned, and "$\beta_{T0}$" is obtained based on the bulk modulus of the oil having the bubbles that is measured beforehand.

As above, the embodiment of the present invention is explained. The scope of the present invention is absolutely defined based on the invention claimed in the claim, and is not limited into the above embodiment.

For example, it is described in the claims that an oil void fraction is measured based on a bulk modulus of the oil having the bubbles; however, obtaining the oil void fraction by measuring an oil compressibility being an inverse number of the bulk modulus of the oil having the bubbles as the above embodiment also belongs to the technical scope of the present invention.

Moreover, in the present embodiment, the valve 3 is mediated in the middle of the engine oil flow path 2, and therefore the valve 3 is connected to the cylinder 1, and by using the cylinder 1, the oil void fraction is measured; however, the oil gathered inside an oil pan for example is extracted by the cylinder (injector), the oil inside the cylinder is sealed, then the piston of the cylinder is depressed so as to obtain the oil compressibility, and the void fraction may be specified.

Moreover, the method of obtaining the oil void fraction easily may be for example a way of deeming to be the oil void fraction by using the ratio "$V_{LO}$" with respect to "$V_{GO}$" simply obtained as above.

## SECOND EMBODIMENT

The below seven premises are used in a method of measuring an oil void fraction of the second embodiment.

Premise 1: The volume change when the bubble-mixed-engine-oil is compressed is equal to the volume change of air included as bubbles. That is, the liquid volume itself is unchanged.
Premise 2: Bubbles included in oil is made of air.
Premise 3: Air is considered to be an ideal gas.
Premise 4: An engine oil evaporation can be ignored.
Premise 5: The pressure inside the bubbles is considered to be equal to the pressure measured by the pressure sensor.
Premise 6: The temperature of air in a sample and the temperature of engine oil are equal from each other.
Premise 7: The compression of the bubble-mixed-engine-oil is performed, keeping the quasi-static state.

At an initial state under a certain temperature, based on the above seven premises, a calibration line is made from sample of bubble-mixed-engine-oil-volume having already-known void fraction, a sample of bubble-mixed-engine-oil having unknown void fraction is compressed, and the void fraction is derived based on the change of the pressure and the volume during that time.

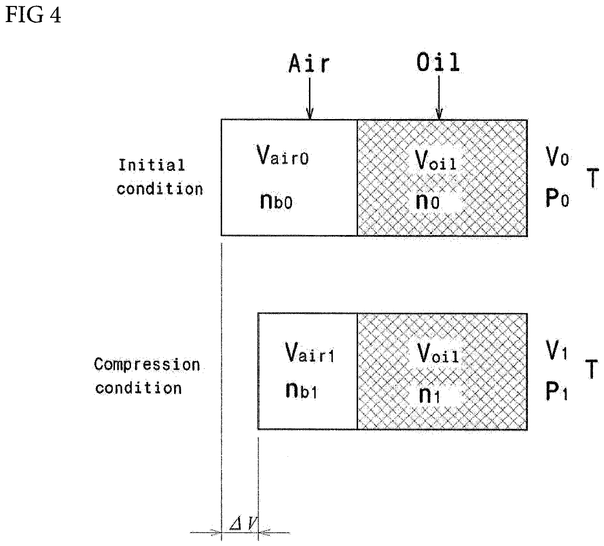

Specifically, based on a concept view of the bubble-mixed-engine-oil in an initial state and a compression-after state (referring to Fig.4), an equation for obtaining the gas state (mathematical formula 4, mathematical formula 5), a physical quantity conservation law (mathematical formula 6), and Henry's law (mathematical formula 7) that are fundamental equations for measurement principle used in the second embodiment are expressed as below. Note that, an air being existed together in a scattering manner as bubbles in engine oil is illustrated in Fig. 4 in a gas phase and a liquid phase separating state.

[mathematical formula 4]

$$P_0 V_{air0} = n_{b0} RT$$

[mathematical formula 5]

$$P_1 V_{air1} = n_{b1} RT$$

[mathematical formula 6]

$$n_{all} = n_{b0} + n_0 = n_{b1} + n_1 = const.$$

[mathematical formula 7]

$$n_1 = \frac{P_1}{P_0} n_0$$

Here, each symbol indicates:

"$V_0$": total volume at an initial state
"$V_1$": total volume in a compression state
"$P_0$": pressure at an initial state
"$P_1$": pressure in a compression state
"T": temperature (even)
"R": gas constant
"$V_{oil}$": engine oil volume
"$V_{air0}$": total volume of bubbles at an initial state
"$V_{air1}$": total volume of bubbles in a compression state
"$n_{all}$": total amount of all the air inside an oil test sample
"$n_{b0}$": total amount of bubbles at an initial state
"$n_{b1}$": total amount of bubbles in a compression state
"$n_0$": total amount of air dissolved in an engine oil at an initial state
"$n_1$": total amount of air dissolved in an engine oil in a compression state
"$\Delta V$": volume change amount

Secondly, the relation of the void fraction, an oil test sample pressure, and volume is considered. If the void fraction at an initial state of an oil test sample is set to be "x" (0<x<1), the engine oil volume "$V_{oil}$" is expressed as follows.

$$V_{oil} = V_0 - V_{air0} = V_0 - x \cdot V_0 = (1 - x) V_0$$

Moreover, if the volume rate of air dissolved in engine oil at the initial state is to be "a" (0<a<1), "$n_0$" is expressed as the below mathematical formula 8.

[mathematical formula 8]

$$n_0 = \frac{P_0 V_0}{RT} (1 - x) a$$

By plugging the formulas from the mathematical formula 6 through 8 into the mathematical formula 5, a state equation of bubbles when an oil test sample is compressed to the pressure "P1" is expressed as the below mathematical formula 9 by using the pressure "$P_0$" at an initial state, an oil test sample volume "$V_0$", the void fraction "x", the volume ratio "a" of the dissolved air in engine oil.

[mathematical formula 9]

$$P_1 V_{air1} = -(1-x)aV_0 P_1 + P_0 V_0 (1-a)x + aP_0 V_0$$

Moreover, if "$P_1 V_{air1}$" is rewritten by volume of an oil test sample at an initial state, the void fraction, and the volume change amount "$\Delta V$" starting from an initial state, "$P_1 V_{air1}$" can be expressed as follows:

$$P_1 V_{air1} = P_1 (V_{air0} - \Delta V) = P_1 (x V_0 - \Delta V)$$

Next mathematical formula 10 can be derived from this formula and the above mathematical formula 9.

[mathematical formula 10]

$$P_1 \Delta V = V_0 \{(1-a)x + a\}P_1 - \{P_0 V_0 (1-a)x + aP_0 V_0\}$$

The right side the first term, the coefficient of "$P_1$" and the other term that is not dependent on "$P_1$" in the mathematical formula 10 constitute a linear equation of void fraction "x". Under a certain temperature and at an initial state, a calibration line is made up from samples of the bubble-mixed-engine-oil-volume having already-known void fraction. Thereby, a sample of the bubble-mixed-engine-oil having unknown void fraction is compressed, and the void fraction of a sample of the bubble-mixed-engine-oil having unknown void fraction can be derived based on the change of the pressure and volume during that time. In below, a preparation of the calibration line based on samples is explained.

The calibration line based on samples is made up by performing experiments (In below, referring to "an oil test sample experiment") by use of a plurality of the void fraction already known oil test samples (In the present embodiment, samples having the void fraction, 17.3%, 23.6%, 30.0% are used) to the system illustrated in Fig. 1.

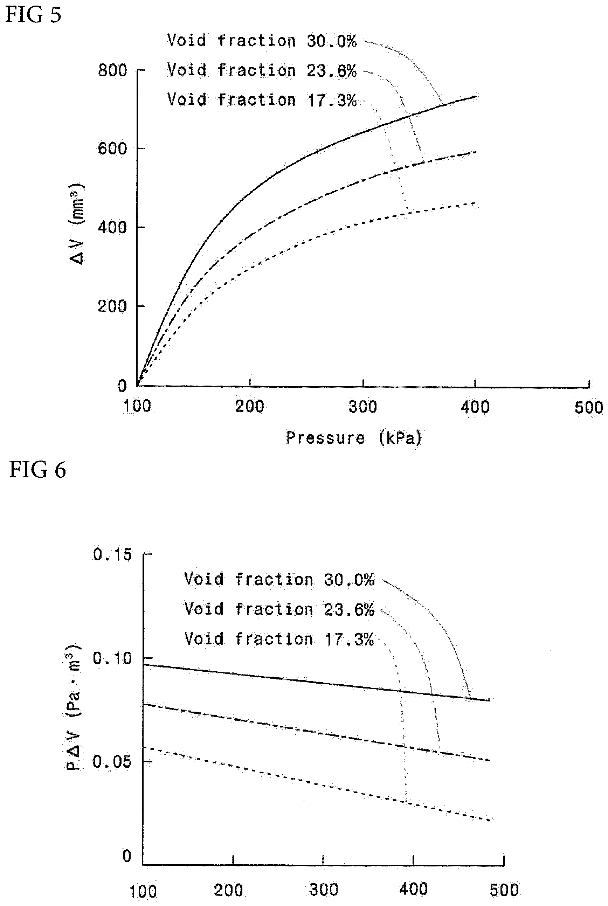

First of all, the compression and the returning to an initial sate is repeated on three kind of samples, and the relation between the in-cylinder pressure and the volume change amount is obtained based on an average among ten times measurement. Fig. 5 illustrates the result thereof, and it can be found out that the larger in size an initial air volume of an oil test sample is, the larger the volume change amount at the same pressure is.

Next, as similar to the case where the relation between the in-cylinder pressure and the volume change amount is obtained, the relation of a product of the pressure and the volume of air that behaves as a gas in an oil test sample against the pressure (In below, referring to "$PV_{air}$") is obtained based on an average among ten times measurement by repeating the compression and the returning to an initial state. Fig. 6 illustrates the result thereof, and it finds out that "$PV_{air}$" of each oil test sample decreases in accordance with pressure increase.

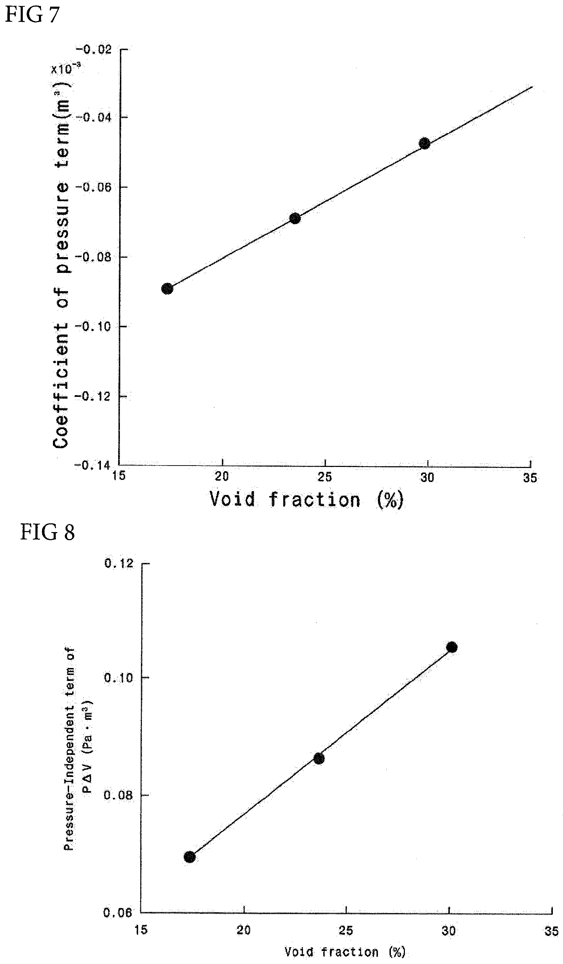

In Fig. 7, respective measurement data illustrated in Fig. 6 are approximated by the least square method as a linear equation of pressure, and Fig. 7 illustrates the relation of "$PV_{air}$" change rate against the pressure of each sample obtained in an average of a coefficient of each "pressure term". In other word, Fig. 7 explains an inclination of each sample illustrated in Fig. 6.

Fig. 8 illustrates a change of a term that is not dependent on pressure of "$PV_{air}$" against pressure of each sample that is obtained by sample examinations. Moreover, the term that is not dependent on the pressure of "$PV_{air}$" can be explained as follows as a function of the void fraction "x" at an initial state from the right side the second term of the mathematical formula 10.

$$f(x) = Ax + B$$

$$A \equiv P_0 V_0 \cdot (1-a)$$

$$B \equiv a \cdot P_0 V_0$$

Therefrom, when "a" and "$P_0V_0$" are found out as follows:

$$a = B/(A+B)$$

$$P_0V_0 = A+B$$

Each value of "A" and "B" obtained by the sample examination is respectively, "A=0.284737", "B=0.01671", the volume rate "a" of air that is dissolved in an engine oil at an initial state is estimated to be about 6 vol %. Similarly, a product of the pressure at an initial state and an oil test sample volume, "$P_0V_0$", becomes about 0.3Pa $\cdot$ m$^3$ and the value is matched with the value having an error somewhat compared to the value that obtained from an initial pressure and an initial volume at the sample examination. Thereby, an existence of a correlative relationship between a value that is obtained from the sample examination result and a value that is obtained by the calculus equation can be affirmed. It is a new insight obtained in the present examination that the volume rate of air "a" that is dissolved in the engine oil at that initial state can be obtained by calculation instantly.

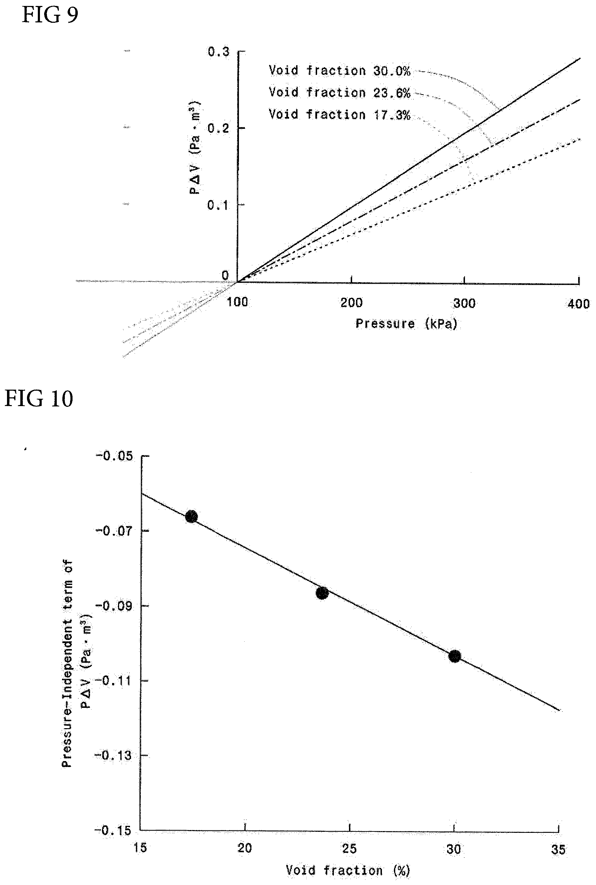

Next, based on the result obtained by the sample examination illustrated in Figs. 5 & 6, the relation of a product of the pressure and the volume change amount (In below, referring to "P$\Delta$V") when it is compressed to the pressure "$P_1$", is shown in Fig.9. It is found out that "P$\Delta$V" is a liner state relationship against the pressure. This is similar to the above, from the right side the second term of the mathematical formula 10, explained as below function of the void fraction "x" at an initial state.

$$g(x) = Cx + D$$

$$C \equiv -P_0V_0 \cdot (1-a)$$

$$B \equiv -a \cdot (1-a)$$

In Fig. 10, this linear equation is illustrated in graph. The figure 10 illustrates the value of "P$\Delta$V" when the pressure of each sample is "0kPa" (the value of y-axis intercept illustrated by fine line of Fig. 9). This becomes a correlation line of the second embodiment. An oil test sample having an unknown void fraction is introduced into the cylinder 1, and a volume change amount ($\Delta$V) when a predetermined pressure (P) is applied is detected. From the detected volume change amount ($\Delta$V), a graph (P$\Delta$V) of a product of the pressure illustrated in Fig. 9 and the volume change amount is made up, the "P$\Delta$V" at "0kPa" is obtained, and thereby, the void fraction is found out instantly from the correlation line illustrated in Fig. 10.

As mentioned above, a method of measuring an oil void fraction of the second embodiment comprises a step of obtaining each oil void fraction of a plurality of sample oils, the void fraction thereof already-known, that are introduced into a closed space, compressing each sample oil with a predetermined pressure and measuring a volume change of the sample oil when compressed, and obtaining a calibration line for each sample oil, that is a linear function by connecting values represented by a product of the pressure at 0kPa and the volume change plotted against the pressure when it is compressed to the predetermined pressure, a step of obtaining a value of a test oil sample having an unknown void fraction, that is represented by a product of the pressure at 0kPa and the volume change plotted against the pressure when it is compressed to the predetermined pressure; and a step of determining the unknown void fraction of the test oil sample by comparing the value of the test sample oil to the calibration line (Fig. 10) of each sample oil.

The above is sent to a controller (the personal computer 7 or microcomputer) from each sensor of an oil void fraction measuring system, a calculation is instantly performed by control program, and unknown oil void fraction can be found out.

## NUMARAL SYMBOLS EXPLANATION

1. Cylinder (Injector)
2. Engine oil flow path

3. Valve
4. Force Sensor
5. Piezo Actuator
6. Piezo Controller
7. Personal Computer
8. Temperature Sensor
11. Piston

## Claims

1. A method of measuring an oil void fraction, comprising:

   a step of introducing an oil having bubbles mixed therewith into a closed space, compressing the oil, and then measuring a bulk modulus of the oil having the bubbles;
   a step of referring to a predetermined oil bulk modulus of the oil which does not have the bubbles, which has been measured beforehand; and
   a step of determining the oil void fraction of the oil having the bubbles based on a ratio of the measured bulk modulus with respect to the referred bulk modulus.

2. A method of measuring an oil void fraction, comprising:

   a step of obtaining each oil void fraction of a plurality of sample oils, the void fraction thereof already-known, that are introduced into a closed space, compressing each sample oil with a predetermined pressure and measuring a volume change of the sample oil when compressed, and obtaining a calibration line for each sample oil, that is a linear function by connecting values represented by a product of the pressure at OkPa and the volume change plotted against the pressure when it is compressed to the predetermined pressure;
   a step of obtaining a value of a test oil sample having an unknown void fraction, that is represented by a product of the pressure at OkPa and the volume change plotted against the pressure when it is compressed to the predetermined pressure; and
   a step of determining the unknown void fraction of the test oil sample by comparing the value of the test sample oil to the calibration line of each sample oil.

FIG 1

1 (cylinder)
11 (piston)
3 (valve)
5 (piezo actuator)
6 (piezo controller)

4 (force sensor)
8 (temperature sensor)

2 (engine oil flow path)

7 (PC)

FIG 2

engine oil

engine oil suction ⟶

bubble

F

Δx

FIG 3

calibration line

FIG 4

FIG 5

FIG 6

FIG 7

FIG 8

FIG 9

FIG 10

## INTERNATIONAL SEARCH REPORT

| International application No. |
| --- |
| PCT/JP2016/082559 |

A. CLASSIFICATION OF SUBJECT MATTER
*G01N7/00*(2006.01)i

According to International Patent Classification (IPC) or to both national classification and IPC

B. FIELDS SEARCHED

Minimum documentation searched (classification system followed by classification symbols)
G01N7/00, G01N33/26

Documentation searched other than minimum documentation to the extent that such documents are included in the fields searched
Jitsuyo Shinan Koho       1922-1996   Jitsuyo Shinan Toroku Koho   1996-2017
Kokai Jitsuyo Shinan Koho   1971-2017   Toroku Jitsuyo Shinan Koho   1994-2017

Electronic data base consulted during the international search (name of data base and, where practicable, search terms used)
JSTPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| Y<br>A | US 2005/0109078 A1  (CHEN et al.),<br>26 May 2005 (26.05.2005),<br>paragraphs [0045] to [0053]<br>& US 6847898 B1        & WO 2005/024351 A2 | 1<br>2 |
| Y<br>A | Takayuki NAKAGAWA, Tsuyoshi OSUMI, "On the bulk modulus of hydraulic fluid under entrained air condition", Bulletin of Faculty of Engineering, Toyama University, 1976, vol.27, pages 25 to 30 | 1<br>2 |
| A | JP 4-230830 A  (Afros S.p.A.),<br>19 August 1992 (19.08.1992),<br>entire text; all drawings<br>& EP 451752 A2 | 1-2 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>21 February 2017 (21.02.17) | Date of mailing of the international search report<br>07 March 2017 (07.03.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

## INTERNATIONAL SEARCH REPORT

| International application No. |
| --- |
| PCT/JP2016/082559 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 56-21036 A  (Polyurethane Engineering Co., Ltd.),<br>27 February 1981 (27.02.1981),<br>entire text; all drawings<br>& US 4329869 A            & DE 3021255 A1 | 1-2 |
| A | Toru KONISHI, "Fundamental properties of lubricating oil (Compressibility of lubricating oil)", Nisseki Technical Review, 1996.11, vol.38, no.4, pages 28 to 35 | 1-2 |
| A | GHOLIZADEH et al., Modeling and Experimental Validation of the Effective Bulk Modulus of a Mixture of Hydraulic Oil and Air, Journal of Dynamic Systems, Measurement, and Control, 2014.09, Vol.136, Pages 051013-1 - 051013-14 | 1-2 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007064820 A

**Non-patent literature cited in the description**

- **SHIMAOKA.** SALD-7100 Nanoparticle Size Analyzer. *Shimazu reviewal, separate print,* 2006, vol. 63th (1, 2
- **MIZUSHIMA et al.** Newly developed method for determining a touch position in sub-millimeter bubble/droplet measurement via a Single-Tip Optical fiber Probe. *Particulate,* 2012, vol. 21 (73
- Bubble behavior measurement in engine lubricant oil. **KIMURA et al.** Academic conference, separate print collection No. 147-08. Automobile Technology Association, 2008
- **NAKAGAWA et al.** On the bulk modulus of hydraulic fluid under entrained air condition. *Bulletin of Faculty of Engineering of Toyama University,* 1976, vol. 27, 25-30